# EUROPÄISCHE PATENTANMELDUNG

(11) **EP 0 936 566 A2**
(43) Veröffentlichungstag der Anmeldung: **18.08.1999**
(21) Anmeldenummer: 99101324.4
(22) Anmeldetag: 25.01.1999
(51) Int. Cl.: G06F 17/60

(54) **System zur Durchführung medizinischer Studien**

(30) Priorität: 11.02.1998 DE 19805535
(71) Anmelder: SIEMENS AKTIENGESELLSCHAFT, 80333 München (DE)
(72) Erfinder: Schmidt, Volker, Dr., 91054 Erlangen (DE); Striebel, Werner, Dipl.-Wirtschaftsmath., 91207 Lauf (DE)

(57) **Zusammenfassung**

System zur Durchführung medizinischer Studien, wobei eine Vielzahl von medizinischen Stellen, wie z.B. Kliniken oder Arztpraxen, über ein Computernetz an einen zentralen Server (2) angeschlossen sind. Das System ist modular aufgebaut, wobei über das System rechnergestützt automatisch festgestellt wird, ob ein bestimmter Patient für die Teilnahme an einer medizinischen Studie des Systems in Frage kommt und, falls ja, automatisch studienspezifisch und patientenspezifisch der Ablauf der entsprechenden medizinischen Studie zentral durch entsprechende Module gesteuert wird.

## Beschreibung

Die vorliegende Erfindung betrifft ein System zur Durchführung medizinischer Studien.

Medizinische Studien werden in der Regel mit Hilfe von Patienten durchgeführt, welche in Kliniken oder Arztpraxen betreut werden. Mit Hilfe derartiger medizinischer Studien kann durch empirische Erhebungen der Erfolg bestimmter Therapiemaßnahmen od. dgl. repräsentativ beurteilt werden. Dabei werden die medizinischen Studien in den Kliniken oder Arztpraxen an bestimmten Patienten durchgeführt, die vorgegebene Einschlußkriterien für die medizinischen Studien erfüllen. Die Ergebnisse der medizinischen Studien werden von einer Studienleitung ausgewertet.

Medizinische Studien, insbesondere Multicenterstudien, erfordern einen hohen Verwaltungs- und Koordinationsaufwand. Bei der Durchführung von medizinischen Studien sind zahlreiche Probleme zu bewältigen. Die an der jeweiligen medizinischen Studie teilnehmenden Kliniken und Arztpraxen müssen über die durchzuführende Studie sowie die entsprechenden Einschlußbzw. Ausschlußkriterien informiert werden. Abhängig von diesen Kriterien müssen Patienten identifiziert bzw. ausgewählt werden, welche für eine Teilnahme an der entsprechenden medizinischen Studie in Frage kommen. Anschließend sind die in Betracht kommenden Patienten bei der Studienleitung anzumelden und geeigneten Studiengruppen zuzuordnen. Schließlich müssen die im Laufe einer medizinischen Studie erhobenen Daten der teilnehmenden Patienten korrekt erfaßt und der Studienleitung mitgeteilt werden.

Bisher erfolgt ein Großteil der Verwaltungsarbeit bei der Durchführung medizinischer Studien durch menschliche Tätigkeit. In der Regel überprüft ein Arzt, ob einer seiner Patienten für eine vorgegebene medizinische Studie in Frage kommt und meldet diesen über mögliche Zwischenstellen der Studienleitung, wobei die Patientendaten in der Regel über Papier übermittelt werden. Die Studienleitung ordnet die Patienten einer geeigneten Studiengruppe zu, erstellt Studienprotokolle, überwacht das rechtzeitige Eingehen von Studiendaten und fordert fehlende Studiendaten an. Die Kommunikation zwischen der Studienleitung und der Arztpraxis bzw. dem teilnehmenden Patienten erfolgt in der Regel über den herkömmlichen Postweg, d.h. mittels Schriftverkehr.

Es ist offensichtlich, daß diese Vorgehensweise einen hohen Verwaltungsaufwand erfordert und demnach zeitintensiv und kostspielig ist. Insbesondere steigt der Verwaltungsaufwand mit der Anzahl der teilnehmenden Patienten bzw. der teilnehmenden Kliniken/Arztpraxen schnell an.

Obgleich bereits Vorschläge für ein Informationssystem zur Durchführung medizinischer Studien über das Internet oder für die Unterstützung der Studienleitung durch Computereinsatz bestehen, sind diese nicht ausreichend spezifiziert, um ein tatsächlich realisierbares Werkzeug für eine computergestützte Studiendurchführung anzugeben (vgl. u.a. 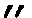 Einsatz von Internet-Diensten und Werkzeugen zur Unterstützung der Dateneingabe und das Monitorings multizentrischer klinischer Studien", P. Wübbelt, C. Eikemeier, H. Hecker, C.-H. Köhne, 42. Jahrestagung der GMDS, Ulm, September 1997, MMV Medizin Verlag München oder 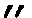 Ein WWW-basiertes Informations- und Kommunikationssystem für multizentrische medizinische Studien", A. Selz, W. Esswein, Vortrag auf der 42. Jahrestagung der GMDS, Ulm, 1997.

Der vorliegenden Erfindung liegt daher die Aufgabe zugrunde, ein System zur Durchführung medizinischer Studien vorzuschlagen, welches eine einfachere und effektivere Durchführung medizinischer Studien ermöglicht.

Diese Aufgabe wird gemäß der vorliegenden Erfindung durch ein System zur Durchführung medizinischer Studien mit den Merkmalen des Anspruches 1 gelöst. Die Unteransprüche beschreiben vorteilhafte und bevorzugte Ausführungsbeispiele der vorliegenden Erfindung, die ihrerseits zu einer Verringerung des Verwaltungsaufwandes und einer effektiven Durchführung der medizinischen Studien beitragen.

Das System gemäß der vorliegenden Erfindung ist durch Computer- bzw. Rechnereinsatz unterstützt. Mehrere Kliniken und/oder Arztpraxen, die zur Teilnahme an medizinischen Studien vorgesehen sind, sind durch ein Computernetzwerk, beispielsweise basierend auf der Internet-Technologie, miteinander über einen zentralen Server verbunden. Dieser zentrale Server dient zur zentralen Verwaltung sämtlicher augenblicklich durchgeführten bzw. durchzuführenden medizinischer Studien sowie insbesondere zur zentralen Steuerung und Kontrolle der augenblicklich durchgeführten Studien. Das erfindungsgemäße System ist ein modular aufgebautes EDV-System, wobei mit Hilfe eines Patientenidentifikationsmoduls anhand vorliegender Patientendaten eines bestimmten Patienten automatisch überprüft wird, ob dieser Patient für eine der medizinischen Studien in Frage kommt. Ist dies der Fall, wird der Patient automatisch von dem zentralen Server der entsprechenden medizinischen Studie zugeordnet und ein auf den zentralen Server implementiertes Studienablaufsteuermodul steuert bzw. kontrolliert zentral durch Kommunikation mit dem Patienten oder der für den Patienten zuständigen medizinischen Stelle den Ablauf der entsprechenden medizinischen Studie.

Über das Computernetz können beispielsweise Arztpraxen aller Fachrichtungen verbunden sein und demnach eine Vielzahl unterschiedlicher Patienten erreicht werden, so daß mit Hilfe des erfindungsgemäßen Systems schnell für durchzuführende medizinische Studien ausreichende Patientenzahlen erzielt werden können. Über den lokalen Rechner, der der entsprechenden Arztpraxis oder Klinik zugeordnet ist, können die behandelnden Ärzte ihre Patienten am zentralen Server anmelden. Durch Überprüfung der entsprechenden Patientendaten wird automatisch festgestellt, ob der jeweilige Patient für eine bestimmte medizinische Studie in Frage kommt. Ist dies der Fall, erhält der anmeldende Arzt beispielsweise eine kurze Information über den Studienaufwand, die Vergütung und dergleichen. Stimmt anschließend der behandelnde Arzt oder der Patient selbst der Teilnahme an der medizinischen Studie zu, werden weitere für die Durchführung der medizinischen Studie relevanten Informationen übermittelt. Nach der Teilnahmezu-Stimmung erfolgt die Studiendurchführung zentral durch den Server des Computernetzes mit Hilfe einer dort implementierten Studiensoftware. Allgemein erfolgt die Kommunikation zwischen der zentralen Studienleitung und den einzelnen Patienten bzw. Ärzten vorteilhafterweise über das Computernetz, d.h. auch die Studiendurchführung erfolgt vorteilhafterweise automatisch durch Übermittlung entsprechender Anfragen an die betreuenden Ärzte oder an die Patienten auf elektronischem Weg.

Vorteilhafterweise sind an das erfindungsgemäß vorgeschlagene Computernetz zur Durchführung medizinischer Studien ambulante Arztpraxen und/oder Kliniken angeschlossen. Dadurch wird gewährleistet, daß die medizinischen Studien vorwiegend in demjenigen Umfeld durchgeführt werden, in dem der Großteil der Arzt-Patientenkontakte und damit der praktischen Medizin stattfindet. Die hohen Fallzahlen im ambulanten Bereich ermöglichen die Zusammenstellung von bezüglich Alter, Multimorbidität usw. sehr gut vergleichbaren Kollektiven bzw. Studiengruppen. Durch den großen Umfang von Patienten im ambulanten Sektor können schnell repräsentative Studienumfänge erreicht und dementsprechend schnell Studienergebnisse erzielt werden. Zwischenergebnisse können über das Computernetz problemlos noch während der Durchführung der entsprechenden Studie jederzeit abgefragt werden. Durch das Ansprechen einer Vielzahl von Kliniken/Arztpraxen unterschiedlicher Fachrichtungen kann eine Vielzahl von Patienten in die einzelnen medizinischen Studien eingebunden und dementsprechend hochqualitative Studien relativ schnell durchgeführt werden. Durch die Vernetzung des zentralen Servers mit den einzelnen lokalen Rechnern, die jeweils einer bestimmten medizinischen Stelle (Arztpraxis, Klinik u. dgl.) zugeordnet sind, ist der organisatorische Aufwand für die einzelnen medizinischen Stellen sowie die Patienten gering.

Gelegentlich sind für an Kliniken durchgeführte Studien Nachuntersuchungen von bereits aus der Studie entlassenen Patienten erforderlich. Mit Hilfe des erfindungsgemäß vorgeschlagenen Systems können diese Patienten leicht von dem zentralen Server über die entsprechenden lokalen Rechner der für die jeweiligen Patienten zuständigen medizinischen Stellen verfolgt und auch nach bereits erfolgter Entlassung aus der medizinischen Studie nochmals angesprochen werden. Für die Klinik gehen daher diese Patienten nicht verloren.

Die Erfindung wird nachfolgend anhand bevorzugter Ausführungsbeispiele unter Bezugnahme auf die beigefügte Zeichnung näher erläutert. Es zeigen:
- Fig. 1: ein vereinfachtes Blockschaltbild eines ersten Ausführungsbeispiels des erfindungsgemäßen Systems zur Durchführung medizinischer Studien, und
- Fig. 2: ein vereinfachtes Blockschaltbild eines zweiten Ausführungsbeispiels des erfindungsgemäßen Systems zur Durchführung medizinischer Studien.

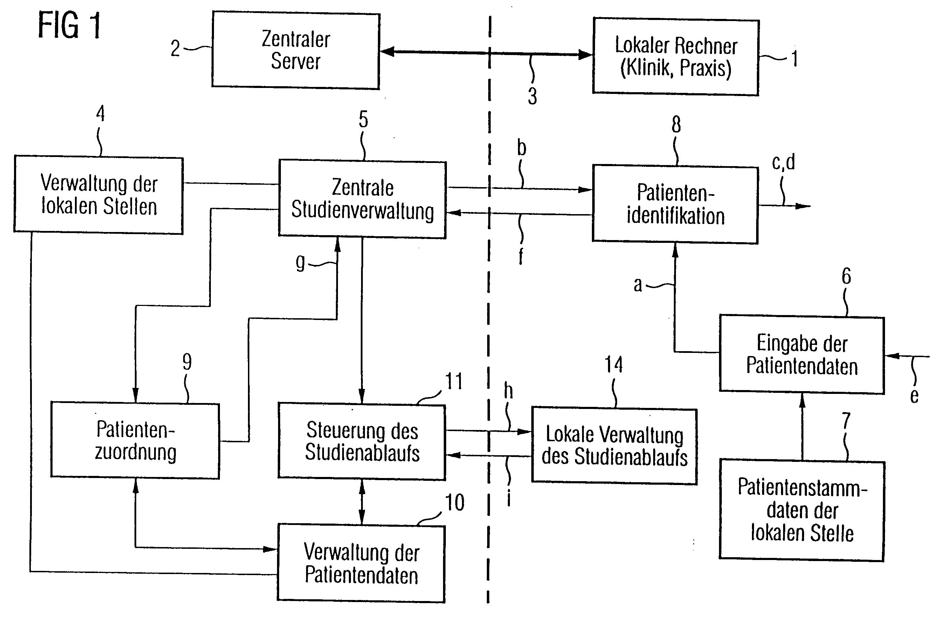

Wie in Fig. 1 gezeigt ist, ist eine Vielzahl von lokalen Rechnern 1 an einen zentralen Server 2 angeschlossen. Der zentrale Server 2 kann mit jedem lokalen Rechner 1 beispielsweise über das herkömmliche Telefonnetz analog oder digital (ISDN) verbunden sein. Die Kommunikation zwischen dem zentralen Server 2 und jedem lokalen Rechner 1 kann über das Internet erfolgen. Jeder lokale Rechner 1 ist einer bestimmten medizinischen Stelle, wie z.B. einer Arztpraxis oder einer Klinik, zugeordnet, welche für die Teilnahme an von dem zentralen Server 2 durchgeführten medizinischen Studien vorgesehen ist.

Das erfindungsgemäße System ist modular aufgebaut, d.h. auf dem zentralen Server 2 sowie jedem lokalen Rechner 1 sind bestimmte Module implementiert, welche im Zusammenspiel für einen reibungslosen Ablauf der einzelnen medizinischen Studien sorgen.

Auf dem zentralen Server 2 ist ein zentrales Studienverwaltungsmodul 5 implementiert. Dieses Studienverwaltungsmodul 5 verwaltet und speichert zentral Informationen über sämtliche durchzuführenden bzw. augenblicklich durchgeführten medizinischen Studien, d.h. alle über das Computernetz durchgeführten medizinischen Studien werden von diesem Studienverwaltungsmodul 5 zentral verwaltet. Dementsprechend erlaubt dieses zentrale Studienverwaltungsmodul 5 auch die Definition jeder einzelnen medizinischen Studie, d.h. die Festlegung des Studienablaufes. In diesem Studienverwaltungsmodul 5 sind studienspezifisch die Ein- bzw. Ausschlußkriterien für jede medizinische Studie hinterlegt. Des Weiteren sind weitere Informationen gespeichert, welche für die Durchführung jeder einzelnen Studie erforderlich sind, wie z.B. der geplante und erreichte Umfang (Teilnehmerzahl) jeder medizinischen Studie oder welche Kontrolluntersuchungen bzw. Therapiemaßnahmen zu welchem Zeitpunkt stattfinden sollen.

Des weiteren ist auf den zentralen Server 2 ein zentrales Modul 4 zur Verwaltung sämtlicher medizinischen Stellen vorgesehen, welche zur Teilnahme an medizinischen Studien berechtigt sind. Das heißt, dieses Modul 4 verwaltet Informationen über die einzelnen Ärzte, Kliniken oder Praxen, die zur Teilnahme an medizinischen Studien vorgesehen sind. Die in den zentralen Modulen 4 und 5 gespeicherten Informationen dienen als Grundlage für die Kommunikation zwischen dem zentralen Server 2 und jedem an das Computernetz angeschlossenen lokalen Rechner, der jeweils einer bestimmten medizinischen Stelle zugewiesen ist.

Neben diesen beiden zentralen Modulen des zentralen Servers 2 sind weitere Module auf dem zentralen Server 2 implementiert, deren Funktion nachfolgend unter Bezugnahme auf die Module der einzelnen lokalen Rechner 1 erläutert werden soll.

Damit beurteilt werden kann, ob ein bestimmter Patient für eine medizinische Studie des Systems in Frage kommt und ggf. dieser medizinischen Studie zugeordnet werden kann, müssen zunächst entsprechende Patientendaten vorliegen, die den jeweiligen Patienten identifizieren und ausreichend beschreiben. Zu diesem Zweck dient ein Patientendateneingabemodul 6, welches auf jedem lokalen Rechner 1 implementiert ist. Dieses Modul 6 dient zur Eingabe/Verwaltung von Daten derjenigen Patienten, die von der entsprechenden medizinischen Stelle des jeweiligen lokalen Rechners 1 betreut werden. Für die Eingabe der Patientendaten sind mehrere Lösungen möglich. So kann das Modul 6 beispielsweise als ein eigenständiger Internet-Dienst implementiert sein, d.h. die entsprechenden Patientendaten jedes einzelnen Patienten werden von dem behandelnden Arzt einzeln zusätzlich zu dem bereits vorhandenen Klinik- bzw. Praxissystem eingegeben und überprüft. Vorteilhafterweise kann diese Eingabe über eine telefonische Abfrage erfolgen, wobei am anderen Ende der Telefonleitung eine Person die erforderlichen Patientendaten abfragt und die Antworten der entsprechenden medizinischen Stelle aufnimmt. Diese Fragen könnten auch computergestützt von einem Computersystem durchgeführt werden, wobei die entsprechende Eingabe der Patientendaten in der entsprechenden medizinischen Stelle über die Telefontastatur erfolgen kann. Diese Möglichkeit gewährleistet, daß lediglich die tatsächlich für die Studien erforderlichen Patientendaten eingegeben und übermittelt werden. Wie in Fig. 1 gezeigt ist, kann das Patientendateneingabemodul 6 jedoch auch vorteilhafterweise mit der Datenverarbeitung der entsprechenden medizinischen Stelle, d.h. mit der bereits bestehenden Patientenstammdatendatei 7 der jeweiligen medizinischen Stelle gekoppelt sein, so daß von dem Patientendateneingabemodul 6 automatisch neue Patienten/Befunde lokal erfaßt und beispielsweise über das Internet an den zentralen Server 2 übermittelt werden können. In diesem Fall würde ein behandelnder Arzt automatisch eine Rückmeldung darüber erhalten, welche seiner Patienten für eine von dem System durchgeführten medizinischen Studie in Betracht kommen, ohne zusätzliche Patientendaten eingeben zu müssen.

Die über das Modul 6 erfaßten Patientendaten werden von einem Patientenidentifikationsmodul 8 überprüft (vgl. Verbindung a). Dieses Patientenidentifikationsmodul überprüft für jede eingehende Patienteninformation, ob der entsprechende Patient für augenblicklich laufende oder durchzuführende Studien des Systems, für die noch Patienten benötigt werden, in Betracht kommt. Zu diesem Zweck greift das Patientenidentifikationsmodul 8 auf die in dem zentralen Studienverwaltungsmodul 5 des zentralen Servers 2 gespeicherten Studieninformationen der einzelnen Studien zu (vgl. Verbindung b) und überprüft anhand der somit gewonnenen Studieninformationen, ob der entsprechende Patient die Einschlußkriterien der jeweiligen Studien erfüllt und nicht bereits Teilnehmer der entsprechenden Studien ist. Reichen die dem Patientenidenfifikationsmodul 8 vorliegenden Patientendaten noch nicht für eine zuverlässige Beurteilung aus, wird von dem Patientenidentifikationsmodul 8 automatisch eine entsprechende Nachfrage oder Rückmeldung an den behandelnden Arzt ausgegeben (vgl. Ausgabe c). Diese Nachfrage kann beispielsweise in Form einer einfachen Rechnermeldung des lokalen Rechners der zuständigen medizinischen Stelle erfolgen. Hat das Patientenidentifikationsmodul 8 an-hand der vorliegenden Patientendaten sowie der von dem zentralen Studienverwaltungsmodul 5 gelieferten Studieninformationen festgestellt, daß der entsprechende Patient für eine der Studien in Betracht kommt, erhält diejenige medizinische Stelle, die die Patientendaten eingegeben hat, automatisch eine Anfrage (z.B. über eine E-Mail-, Internet- oder Faxmeldung), ob der behandelnde Arzt und der entsprechende Patient an der Studie teilnehmen (vgl. Ausgabe d). Dabei können für die Teilnahme erforderliche Dokumente (z.B. eine Einverständniserklärung) automatisch an die zuständige medizinische Stelle geschickt bzw. von dem zuständigen lokalen Rechner 1 ausgegeben werden.

Durch eine entsprechende Mitteilung kann nunmehr der zuständige Arzt bzw. der betroffene Patient die Teilnahme an der vorgeschlagenen Studie bestätigen und genehmigen. Dies kann insbesondere in Form einer Eingabe an dem zuständigen lokalen Rechner 1 der medizinischen Stelle erfolgen (vgl. Eingabe e).

Liegt eine Teilnahmeerklärung des Patienten vor, wird der Patient in die vorgeschlagene medizinische Studie aufgenommen und die Patientendaten dem zentralen Server 2 endgültig zur Verfügung gestellt (vgl. Verbindung f). Bei dem in Fig. 1 gezeigten Ausführungsbeispiel ist auf jedem lokalen Rechner 1 ein Patientenidentifikationsmodul 8 implementiert. Auf diese Weise wird sichergestellt, daß nicht unnötig Patientendaten an den zentralen Server 2 übermittelt werden, obwohl der entsprechende Patient noch nicht seine Teilnahme an der vorgeschlagenen medizinischen Studie bestätigt hat. Selbstverständlich kann das Patientenidentifikationsmodul 8 jedoch auch zentral auf den zentralen Server 2 implementiert sein.

Nach der Teilnahmeerklärung des behandelnden Arztes sowie des teilnehmenden Patienten werden die Patientendaten sowie weitere erforderliche Informationen, z.B. betreffend den behandelnden Arzt, einem Patientenzuordnungsmodul 9 zugewiesen. Dieses Modul 9 sorgt dafür, daß ein an einer medizinischen Studie teilnehmender Patient einer entsprechenden Studiengruppe für diese medizinische Studie zugewiesen wird. Vorteilhafterweise erfolgt dabei die Zuordnung derart, daß möglichst vergleichbare und gleichermaßen aussagekräftige Studiengruppen von dem Patientenzuordnungsmodul 9 erzeugt werden.

Zu diesem Zweck greift das Patientenzuordnungsmodul 9 auf die Daten eines weiteren zentral implementierten Patientendatenverwaltungsmoduls 10 zu, in dem studienspezifisch sämtliche Daten aller an den einzelnen medizinischen Studien teilnehmenden Patienten verwaltet werden. Auf diese Weise kann das Patientenzuordnungsmodul 9 einen Patienten einer Studiengruppe automatisch unter Berücksichtigung der anderen Teilnehmer der betroffenen Studie zuordnen.

Nachdem durch das Patientenzuordnungsmodul 9 der Patient einer Studiengruppe der medizinischen Studie zugeordnet worden ist, erfolgt durch eine entsprechende Mitteilung eine automatische Aktualisierung der in dem zentralen Studienverwaltungsmodul 5 gespeicherten Studiendaten (vgl. Mitteilung g), um insbesondere dort die Informationen bezüglich der Teilnehmerzahl der entsprechenden Studie auf den neuesten Stand zu bringen.

Wie bereits erläutert worden ist, dient das Patientendatenverwaltungsmodul 10 zur zentralen Verwaltung sämtlicher Daten der an den einzelnen Studien des Systems teilnehmenden Patienten. Insbesondere dient dieses Modul 10 studien- und patientenspezifisch zur Verwaltung der im Rahmen der jeweiligen Studien erhobenen Daten der einzelnen Teilnehmer.

Von zentraler Bedeutung ist schließlich das ebenfalls auf dem zentralen Server 2 implementierte Studienablaufsteuermodul 11. Dieses Modul 11 dient zur Steuerung bzw. Kontrolle des Studienablaufes jedes einzelnen Patienten. Zu diesem Zweck greift das Studienablaufsteuermodul 11 einerseits auf die in dem zentralen Studienverwaltungsmodul 5 gespeicherten Informationen über die einzelnen Studien sowie auf die in dem Patientendatenverwaltungsmodul 10 gespeicherten Patientendaten der einzelnen Patienten zu, um anhand der Studienbeschreibungsdaten sowie der Patientendaten studien- und patientenspezifisch jederzeit festzustellen, welche Maßnahmen bei dem einzelnen Teilnehmer bzw. Patienten aktuell zu treffen sind.

Für jeden Patienten werden von diesem Studienablaufsteuermodul 11 studien- und zeitabhängig automatisch Anfragen an die betreuenden medizinischen Stellen oder direkt an den Patienten verschickt, die Informationen darüber enthalten, welche Kontrolluntersuchungen oder Therapiemaßnahmen demnächst durchzuführen sind (vgl. Ausgabe h). Diese Mitteilungen bzw. Anfragen können von dem zentralen Server 2 beispielsweise per Faxmitteilung an die zuständige medizinische Stelle gesendet werden. Da jedoch der zentrale Server 2 ohnehin über das Computernetz 3 mit den einzelnen lokalen Rechnern 1 der medizinischen Stellen verbunden ist, ist eine Ausgabe dieser Informationen über den elektronischen Weg (z.B. per E-Mail, Internet u. dgl.) vorteilhaft. Auf jedem lokalen Rechner 1 kann ein lokales Verwaltungsmodul 14 implementiert sein, welches die eingehenden Anfragen bzw. Mitteilungen des zentralen Studienablaufsteuermoduls 11 verwaltet und ausdruckt.

Die von dem Studienablaufsteuermodul 11 versandten Mitteilungen erfordern in der Regel entsprechende Rückmeldungen seitens der zuständigen medizinischen Stelle, die über das Ergebnis der durchzuführenden Kontrolluntersuchungen oder Therapiemaßnahmen Aufschluß geben und für die Auswertung der entsprechenden medizinischen Studie von dem zentralen Server 2 verwendet werden können. Diese Rückmeldungen können im Prinzip auf demselben Weg wie die Meldungen des Studienablaufsteuermoduls 11 übermittelt werden (vgl. in Fig. 1 die Rückmeldungen e). Das Studienablaufsteuermodul 11 überwacht automatisch den Eingang der angeforderten Informationen und führt beispielsweise bei Nichteinhaltung einer vorgegebenen zeitlichen Frist automatisch zu einer Anmahnung der fehlenden Informationen bei der zuständigen medizinischen Stelle sowie einer entsprechenden Benachrichtigung an die Studienleitung. Untersuchungsdaten, die durch Rückmeldungen der zuständigen medizinischen Stellen von dem zentralen Studienablaufsteuermodul 11 im Rahmen medizinischer Studien gewonnen werden, werden automatisch in dem Patientendatenverwaltungsmodul 10 abgespeichert.

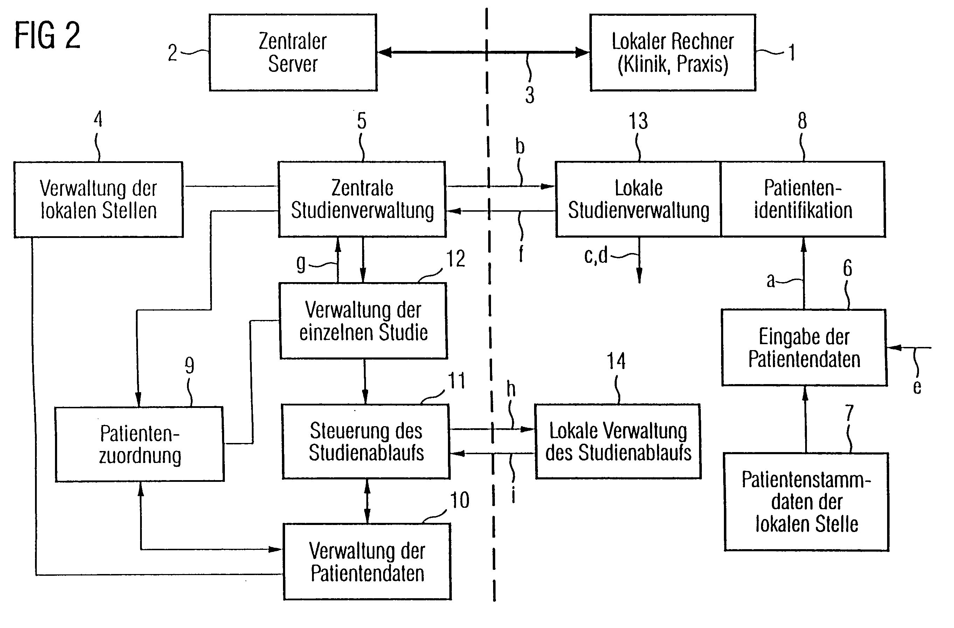

Fig. 2 zeigt ein zweites Ausführungsbeispiel des erfindungsgemäßen Systems zur Durchführung medizinischer Studien, wobei das in Fig. 2 gezeigte System im wesentlichen dem in Fig. 1 gezeigten System entspricht. Zusätzlich ist jedoch auf dem zentralen Server 2 ein zentrales Einzelstudienverwaltungsmodul 12 implementiert, welches als Schnittstelle zwischen dem Patientenzuordnungsmodul 9 und dem Studienablaufsteuermodul 11 einerseits sowie dem zentralen Studienverwaltungsmodul 5 andererseits dient. Dieses zusätzliche Modul 12 speichert demnach die für jede einzelne medizinische Studie des Systems erforderlichen Daten und vereint demnach Funktionen, die bei dem in Fig. 1 gezeigten Ausführungsbeispiel zum Teil von dem zentralen Studienverwaltungsmodul 5 und dem Studienablaufsteuermodul 11 wahrgenommen werden. Des weiteren ist gemäß Fig. 2 jedes Patientenidentifikationsmodul 8 mit einem optionalen lokalen Studienverwaltungsmodul 13 gekoppelt, welches als Schnittstelle zu dem zentralen Studienverwaltungsmodul 5 des zentralen Servers 2 dient. Dieses lokale Studienverwaltungsmodul 13 dient demnach zur Übertragung von Patientendaten zwischen dem entsprechenden lokalen Rechner 1 und dem zentralen Server 2 und übernimmt bestimmte Funktionen, die bei dem in Fig. 1 gezeigten Ausführungsbeispiel von dem Patientenidentifikationsmodul 8 wahrgenommen werden.

Das in Fig. 1 und 2 gezeigte erfindungsgemäße System ermöglicht somit eine automatische Beurteilung von vorliegenden Patientendaten in Bezug auf eine mögliche Aufnahme in eine von dem System durchzuführende medizinische Studie. Die Verarbeitung der Patientendaten erfolgt rechnergestützt und die Studiendaten der einzelnen teilnehmenden Patienten werden automatisch erfaßt, wobei das System mit Hilfe des Studienablaufsteuermoduls 11 abhängig von den vorliegenden Informationen über die durchzuführende Studie und den vorliegenden Patientendaten des teilnehmenden Patienten selbständig über die nächsten einzuholenden Informationen oder die zu treffenden Maßnahmen entscheidet. Insgesamt ist somit mit Hilfe des erfindungsgemäßen Systems nicht nur die Erfassung bzw. die Teilnahme einer Vielzahl unterschiedlicher Patienten an medizinischen Studien gewährleistet, sondern die bei der Durchführung der einzelnen medizinischen Studien gewonnenen Studiendaten können äußerst einfach und ohne großen verwaltungstechnischen Aufwand verarbeitet und ausgewertet werden.

## Patentansprüche

1. System zur Durchführung medizinischer Studien,
wobei mehrere lokale Rechner (1) mit einem zentralen Server (2) zu einem Rechnernetz verbunden und jeweils einer bestimmten medizinischen Stelle zugeordnet sind, welche zur Teilnahme an einer medizinischen Studie des Systems vorgesehen ist,
mit einem auf dem zentralen Server (2) implementierten Studienverwaltungsmodul (5), welches Studieninformationen über sämtliche augenblicklich durchgeführten und durchzuführenden medizinischen Studien verwaltet,
mit einem Patientenidendifikationsmodul (8), welches bei Vorliegen von Patientendaten eines bestimmten Patienten einer der medizinischen Stellen durch Auswerten der Studieninformationen des Studienverwaltungsmoduls (5) anhand der Patientendaten beurteilt, ob der bestimmte Patient für die Teilnahme an einer der Studien des Systems in Betracht kommt, und
mit einem auf dem zentralen Server (2) implementierten Studienablaufsteuermodul (11), welches nach Feststellen des Patientenidentifikationsmoduls (8), daß der bestimmte Patient für eine bestimmte medizinische Studie des Systems in Betracht kommt, durch Kommunikation mit der für den bestimmten Patienten zuständigen medizinischen Stelle den Ablauf der bestimmten medizinischen Studie steuert und dabei entsprechende Studiendaten erhebt.

2. System nach Anspruch 1,
**dadurch gekennzeichnet**, daß die den medizinischen Stellen zugeordneten lokalen Rechner (1) mit dem zentralen Server (2) über das Telefonnetz verbunden sind.

3. System nach Anspruch 1 oder 2,
**dadurch gekennzeichnet**, daß auf dem zentralen Server (2) ein Stellenverwaltungsmodul (4) implementiert ist, welches Informationen über alle medizinischen Stellen verwaltet, die den an den zentralen Server (2) angeschlossenen lokalen Rechnern (1) zugeordnet sind.

4. System nach einem der vorhergehenden Ansprüche, **dadurch gekennzeichnet**, daß auf dem zentralen Server (2) ein Teilnehmerdatenverwaltungsmodul (10) implementiert ist, welches studienspezifisch Teilnehmerdaten sämtlicher an einer medizinischen Studie des Systems teilnehmenden Patienten verwaltet.

5. System nach Anspruch 4,
**dadurch gekennzeichnet**, daß das Teilnehmerdatenverwaltungsmodul (10) patientenspezifisch die im Rahmen einer medizinischen Studie von dem Studienablaufsteuermodul (11) erhobenen Daten der an der entsprechenden medizinischen Studie teilnehmenden Patienten speichert.

6. System nach einem der vorhergehenden Ansprüche,
**dadurch gekennzeichnet**, daß auf dem zentralen Server (2) ein Patientenzuordnungsmodul (9) implementiert ist, welches bei Teilnahme eines bestimmten Patienten an einer bestimmten medizinischen Studie des Systems den bestimmten Patienten einer Studiengruppe der bestimmten medizinischen Studie zuordnet.

7. System nach Anspruch 6 und einem der Ansprüche 4 oder 5, **dadurch gekennzeichnet**, daß das Patientenzuordnungsmodul (9) einen teilnehmenden Patienten abhängig von den in dem Teilnehmerdatenverwaltungsmodul (10) gespeicherten Teilnehmerdaten der an der bestimmten Studie bereits teilnehmenden Patienten einer Studiengruppe der bestimmten medizinischen Studie derart zuordnet, daß die für die bestimmte medizinische Studie gebildeten Studiengruppen weitgehend vergleichbar sind.

8. System nach einem der vorhergehenden Ansprüche,
**dadurch gekennzeichnet**, daß das Patientenidentifikationsmodul (8) auf jedem an dem zentralen Server (2) angeschlossenen lokalen Rechner (1) implementiert ist.

9. System nach einem der vorhergehenden Ansprüche,
**dadurch gekennzeichnet**, daß das Studienverwaltungsmodul (5) studienspezifisch Ablaufinformationen über den zeitlichen Ablauf jeder medizinischen Studie des Systems, Bedingungen für die Teilnahme eines Patienten an einer bestimmten medizinischen Studie des Systems und Informationen über die augenblickliche Teilnehmerzahl jeder medizinischen Studie des Systems speichert.

10. System nach Anspruch 9,
**dadurch gekennzeichnet**, daß das Patientenidentifikationsmodul (8) anhand der in dem Studienverwaltungsmodul (5) gespeicherten Bedingungen für die einzelnen medizinischen Studien des Systems sowie den vorliegenden Patientendaten eines bestimmten Patienten beurteilt, ob der bestimmte Patient für die Teilnahme an einer bestimmten medizinischen Studie des Systems in Betracht kommt.

11. System nach Anspruch 10,
**dadurch gekennzeichnet**, daß das Patientenidentifikationsmodul (8) feststellt, daß der bestimmte Patient für die Teilnahme an einer bestimmten medizinischen Studie des Systems in Betracht kommt und eine entsprechende Mitteilung ausgibt, falls die Patientendaten des bestimmten Patienten die Bedingungen der bestimmten medizinischen Studie erfüllen und der bestimmte Patient noch nicht Teilnehmer der bestimmten medizinischen Studie ist.

12. System nach einem der vorhergehenden Ansprüche,
**dadurch gekennzeichnet**, daß das Patientenidentifikationsmodul (8) eine entsprechende Fehlermeldung ausgibt, falls die zur Verfügung stehenden Patientendaten eines Patienten nicht für die Beurteilung ausreichen, ob der Patient für die Teilnahme an einer der medizinischen Studien des Systems in Betracht kommt oder nicht.

13. System nach einem der vorhergehenden Ansprüche,
**dadurch gekennzeichnet**, daß auf jedem lokalen Rechner (1) ein Patientendateneingabemodul (6) zur Eingabe und Verwaltung der Patientendaten von Patienten, für die die dem jeweiligen lokalen Rechner (1) zugeordnete medizinische Stelle zuständig ist, vorgesehen ist.

14. System nach Anspruch 13,
**dadurch gekennzeichnet**, daß das Patientendateneingabemodul (6) auf jedem lokalen Rechner (1) mit einer Patientenstammdatendatei (7) der dem jeweiligen lokalen Rechner (1) zugeordneten medizinischen Stelle gekoppelt ist und die Patientendaten aus dieser Patientenstammdatendabei (7) automatisch einliest und dem Patientenidentifikationsmodul (8) zur Verfügung stellt.

15. System nach Anspruch 13,
**dadurch gekennzeichnet**, daß das Patientendateneingabemodul (6) auf jedem lokalen Rechner (1) derart ausgestaltet ist, daß die Patientendaten eines Patienten extra an dem entsprechenden lokalen Rechner (1) einzugeben sind.

16. System nach Anspruch 15,
**dadurch gekennzeichnet**, daß das Patientendateneingabemodul (6) auf jedem lokalen Rechner (1) derart ausgestaltet ist, daß die Patientendaten über eine telefonische Abfrage einzugeben sind.

17. System nach Anspruch 16,
**dadurch gekennzeichnet**, daß die Patientendaten über eine Telefontastatur eingebbar sind.

18. System nach einem der vorhergehenden Ansprüche,
**dadurch gekennzeichnet**, daß das Studienablaufsteuermodul (11) anhand der Patientendaten eines bestimmten Patienten und der Studieninformationen über eine bestimmte Studie den zeitlichen Ablauf der bestimmten Studie für den bestimmten Patienten steuert.

19. System nach einem der vorhergehenden Ansprüche,
**dadurch gekennzeichnet**, daß das Studienablaufsteuermodul (11) den zeitlichen Ablauf jeder medizinischen Studie des Systems durch Mitteilungen an diejenige medizinische Stelle steuert, deren Patienten Teilnehmer an der entsprechenden medizinischen Studie sind.

20. System nach Anspruch 19,
**dadurch gekennzeichnet**, daß die Mitteilungen studienspezifisch und abhängig vom augenblicklichen Stand der entsprechenden Studie automatisch von dem Studienablaufsteuermodul (11) erzeugt und an die betroffene medizinische Stelle versendet werden.

21. System nach Anspruch 20,
**dadurch gekennzeichnet**, daß die Mitteilungen von dem Studienablaufsteuermodul (11) elektronisch über das Rechnernetz an die betroffenen medizinischen Stellen versendet werden.

22. System nach einem der Ansprüche 19 bis 21,
**dadurch gekennzeichnet**, daß das Studienablaufsteuermodul (11) den Eingang von Rückmeldungen von den betroffenen medizinischen Stellen als Antwort auf die versandten Mitteilungen überwacht und bei Fehlen einer derartigen Rückmeldung eine entsprechende Fehlermeldung erzeugt.

23. System nach einem der vorhergehenden Ansprüche,
**dadurch gekennzeichnet**, daß das Studienablaufsteuermodul (11) den Ablauf der bestimmten Studie des bestimmten Patienten nur steuert, falls nach Feststellung des Patientenidentifikationsmoduls (8), daß der bestimmte Patient für die bestimmte Studie des Systems in Betracht kommt, zudem für die Teilnahme an der bestimmten medizinischen Studie eine entsprechende Genehmigung der für den bestimmten Patienten zuständigen medizinischen Stelle oder des bestimmten Patienten selbst vorliegt.
